# EUROPÄISCHE PATENTSCHRIFT

(11) **EP 2 797 669 B1**
(45) Veröffentlichungstag und Bekanntmachung des Hinweises auf die Patenterteilung: **04.04.2018**
(21) Anmeldenummer: 12766032.2
(22) Anmeldetag: 17.09.2012
(51) Int. Cl.: A61Q 5/10, A61Q 5/06, A61K 8/37, A61K 8/81, A61K 8/42, A61Q 5/00, A61K 8/40, A61K 8/60, A61Q 5/12

(54) **PFLEGENDE FÄRBEMITTEL FÜR KERATINISCHE FASERN**
CONDITIONING DYEING AGENT FOR KERATINOUS FIBRES
COLORANTS SOINS POUR FIBRES KÉRATINIQUES

(30) Priorität: 20.09.2011 DE 102011083021
(43) Veröffentlichungstag der Anmeldung: 05.11.2014
(73) Patentinhaber: Henkel AG & Co. KGaA, 40589 Düsseldorf (DE)
(72) Erfinder: WITTE, Christiane, 25491 Hetlingen (DE); SCHWARTZ, Stephan, 22880 Wedel (DE)
(86) Internationale Anmeldenummer: PCT/EP2012/068244
(87) Internationale Veröffentlichungsnummer: WO 2013/041485

(56) Entgegenhaltungen:
- EP-A1- 1 555 010
- WO-A1-01/47480
- WO-A1-97/47282
- WO-A1-2005/082321
- DE-A1- 10 163 860
- FR-A1- 2 849 774
- Jörg Kahre: "APG: Innovative Surfactants Made From Sugar and Fat", Skin Care Forum, 1. Juli 1995 (1995-07-01), XP055131785, Düsseldorf, Germany Gefunden im Internet: URL:http://www.skin-care-forum.basf.com/do cs/archivausgaben-1-20/scf12_gb_may1995.pd f?sfvrsn=2 [gefunden am 2014-07-28]
- JESSICA CECCHINI ET AL: "Silicone Alternatives in Personal Care", INTERNET CITATION, November 2010 (2010-11), pages 1-3, XP002687906, Retrieved from the Internet: URL:http://www.happi.com/articles/2010/11/ silicone-alternatives-in-personal-care [retrieved on 2012-11-22]
- DATABASE GNPD [Online] MINTEL; 1 June 2011 (2011-06-01), Schwarzkopf & Henkel: "Leave-in Mask", Database accession no. 1582658
- DATABASE GNPD [Online] MINTEL; 1 August 2011 (2011-08-01), Joico Laboratories: "Treatment Masque", Database accession no. 1605921
- DATABASE GNPD [Online] MINTEL; 1 July 2011 (2011-07-01), Schwarzkopf & Henkel: "Intensive Cream Colour", Database accession no. 1570896

## Beschreibung

Die Erfindung betrifft Färbemittel für keratinhaltige Fasern für intensive, langanhaltende Färbeergebnisse bei verbessertem Pflegezustand der keratinischen Fasern.

Zur modischen Farbgestaltung von Frisuren oder zur Kaschierung von ergrautem oder gar weißem Haar mit modischen oder natürlichen Farbtönen greift der Verbraucher zu farbverändernden Mitteln. Diese Mittel sollen neben der gewünschten Farbveränderung möglichst minimale Schädigungen auf dem Haar hervorrufen, vorzugsweise sogar zusätzliche Pflegeeigenschaften besitzen und das Haar in einem optisch ansprechenden Zustand versetzen.

Zur Bereitstellung färbender kosmetischer Mittel, insbesondere für die Haut oder keratinhaltige Fasern wie beispielsweise menschliche Haare, kennt der Fachmann je nach Anforderungen an die Farbveränderung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, die unter dem Einfluss von Oxidationsmitteln oder von Luftsauerstoff untereinander die eigentlichen Farbstoffe ausbilden. Die Oxidationsfärbemittel zeichnen sich durch hervorragende, lang anhaltende Färbeergebnisse aus. Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Substrat aufziehen und keinen oxidativen Prozess zur Ausbildung der Farbe benötigen. Schließlich werden Färbesysteme auf Basis von Vorstufen naturanaloger Farbstoffe vertrieben, bei denen die Vorläuferverbindungen, vorwiegend unter Einfluss von Luftsauerstoff, miteinander reagieren und dabei den natürlichen Melanin-Farbstoffen strukturell ähnliche, farbige Makromoleküle ausbilden. Nach der Farbveränderung, insbesondere nach einer oxidativen Farbveränderung verbleibt das Haar mit jedoch häufig in einem haptisch und optisch wenig ansprechenden Zustand.

Aufgabe der vorliegenden Erfindung ist es daher, die oben genannten Nachteile von handelsüblichen Haarfärbemitteln herabzusenken. Die bereitzustellenden Farbveränderungsmittel sollen die Faserstruktur möglichst nur minimal schädigen und dem Haar eine erhöhte Elastizität und Geschmeidigkeit und insbesondere einen verbesserte Kämmbarkeit verleihen. Bevorzugt soll dies jedoch nicht zu Lasten einer verringerten Farbveränderungsleistung der Mittel erreicht werden. Insbesondere sollen die Färbemittel glänzende, leuchtende, lang anhaltende und homogene Färbungen erzielen.

WO 2001/047480A1 beschäftigt sich mit Haarkonditioniermitteln, insbesondere mit der verbesserten Haarkonditionierung durch die Kombination von Dialkylcarbonaten mit einem Amid-Rohstoff wie Tegoamid S 18. WO 2005/082321A1 beschäftigt sich mit der Verbesserung des Farberhalts und der Waschbeständigkeit von Haarfärbungen durch kationische Stärke.

Im Zuge ihrer Untersuchungen hat die Anmelderin nun gefunden, dass insbesondere durch eine spezifische Kombination von Inhaltsstoffen in der Trägerbasis von Färbemitteln für keratinische Fasern einen positiven Einfluss auf Pflegeleistung und Schädigungsgrad der Fasern einerseits und auf die Färbeergebnisse andererseits ausübt. Dabei werden neben einem verbesserten Pflegezustand Färbeergebnisse ermöglicht, die den gängigen Handelsprodukten ebenbürtig oder gar überlegen sind.

Ein erster Gegenstand der Erfindung ist daher ein Mittel zum Färben keratinischer Fasern, welches in einem kosmetisch akzeptablen Träger mindestens einen direktziehenden Farbstoff, dadurch gekennzeichnet, dass das Mittel eine Trägerbasis-Kombination aus
(i) mindestens einem Fettkörper (A) der Formel (I), 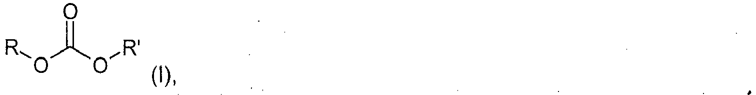 worin R und R' jeweils unabhängig voneinander für eine gesättigte oder ungesättigte C₆-C₂₂-Alkylkette stehen,
(ii) mindestens einem nichtionischen Tensid (B) der Formel (II),

   R1-O-[G]ₚ (II),

   worin R1 für eine gesättigte oder ungesättigte C₆-C₂₂-Alkylkette, G für einen Zuckerrest mit 5 oder 6 Kohlenstoffatomen und p für eine Zahl von 1 bis 10 stehen und
(iii) mindestens einem kationischen Polymer (C)
enthält, dadurch gekennzeichnet, dass das Mittel als kationisches Polymer (C) Polyquaternium-37 enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die zur erfindungsgemäßen Verwendung eingesetzten Zubereitungen enthalten die Wirkstoffe in einem kosmetischen akzeptablen Träger. Dieser kosmetische Träger ist im Sinne der Erfindung wässrig, alkoholisch oder wässrig-alkoholisch. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Bevorzugte Träger stellen dabei Emulsionen und Gele dar, wobei Emulsionen besonders bevorzugt sind.

Als wesentlichen Inhaltsstoff enthält das erfindungsgemäße Mittel mindestens einen direktziehenden Farbstoff.

Die erfindungsgemäßen Mittel enthalten mindestens einen direktziehenden Farbstoff. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe können in anionische, kationische und' nichtionische direktziehende Farbstoffe unterteilt werden. Die direktziehenden Farbstoffe werden jeweils bevorzugt in einer Menge von 0,001 bis 20 Gew.-%, insbesondere von 0,05 bis 5 Gew.-%, jeweils bezogen auf die gesamte Anwendungszubereitung, eingesetzt. Die Gesamtmenge an direktziehenden Farbstoffen beträgt vorzugsweise höchstens 20 Gew.-%.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen. Bevorzugte kationische direktziehende Farbstoffe sind dabei kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie Basic Yellow 57, Basic Red 76, Basic Blue 99, Basic Brown 16 und Basic Brown 17 und HC Blue 16, sowie Basic Blue 347, Basic Yellow 87, Basic Orange 31 und Basic Red 51. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Als weiteres Merkmal enthalten die erfindungsgemäßen Mittel eine Trägerbasis-Kombination aus
(i) mindestens einem Fettkörper (A) der Formel (I), 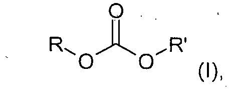
(ii) mindestens einem nichtionischen Tensid (B) der Formel (II) und
(iii) mindestens einem kationischen Polymer (C), wobei als kationisches Polymer Polyquaternium-37 enthalten ist.

Als Fettkörper (A) werden Verbindungen der Formel (I) eingesetzt, in der R und R' jeweils unabhängig voneinander für eine gesättigte oder ungesättigte C₆-C₂₀-Alkylkette stehen.
Die C₆-C₂₂-Alkylkette kann dabei unverzweigt sein oder Verzweigungen aufweisen, insbesondere wenn sie sich von Guerbet-Alkoholen ableitet. Beispiele für erfindungsgemäße C₆-C₂₂-Alkylketten sind n-Hexyl, n-Heptyl, n-Octyl, n-Nonyl, n-Decyl, i-Decyl, n-Dodecyl (Lauryl), n-Tridecyl, i-Tridecyl, n-Tetradecyl (Myristyl), n-Pentadecyl, n-Hexadecyl (Cetyl), n-Heptadecyl, n-Octadecyl (Stearyl), n-Eicosyl (Arachyl), n-Docosyl (Behenyl), 2-Ethylhexyl, 2-Butyloctyl, 2-Hexyldecyl, 2-Octyldodecyl, 2-Heptylundecyl sowie Oleyl, Linolenyl, Linoleyl, Palmitoleyl, Erucyl und Brassidyl. Es kann erfindungsgemäß bevorzugt sein, Verbindungen auf Basis von Alkylkettengemischen einzusetzen, insbesondere wenn die Alkylketten herstellungsbedingt als Gemische anfallen. Beispiele sind Cetearyl (C₁₆/C₁₈-Gemisch), Cocosalkyl (Alkylkettengemisch aus dem Fettsäureschnitt von Cocosfett), Olivalkyl (Alkylkettengemisch aus dem Fettsäureschnitt von Olivenöl) oder Talgalkyl/Tallow Alkyl (Alkylkettengemisch aus dem Fettsäureschnitt von Talg).
In bevorzugten Fettkörpern (A) der Formel (I) stehen R und R' jeweils unabhängig voneinander für eine gesättigte oder ungesättigte C₆-C₁₄-Alkylkette, bevorzugt eine C₆-C₁₀-Alkylkette.
Eine Ausführungsform des ersten Erfindungsgegenstands ist dadurch gekennzeichnet, dass das Mittel als bevorzugte Fettkörper der Formel (I) Di-n-octylcarbonat und/oder Di-(2-Ethylhexyl)carbonat enthält.

Bevorzugt enthält das Mittel einen Gesamtanteil an Fettkörpern (A) von 0,05 bis 8,5 Gew.-%, bevorzugt von 0,1 bis 6 Gew.-%, insbesondere von 0,2 bis 4 Gew.-% und weiter bevorzugt von 0,3 bis 2,5 Gew.-%, jeweils bezogen auf das Gesamtgewicht des Mittels.

Als weiteren wesentlichen Inhaltsstoff enthält das Mittel mindestens ein nichtionisches Tensid (B) der Formel (II),

R1-O-[G]ₚ (II),

worin R1 für eine gesättigte oder ungesättigte C₆-C₂₂-Alkylkette, G für einen Zuckerrest mit 5 oder 6 Kohlenstoffatomen und p für eine Zahl von 1 bis 10 stehen.

Verbindungen der Formel (II) im Sinne der Erfindung sind insbesondere Alkylpolyglucoside. Der Zuckerrest G leitet sich dabei von Aldosen oder Ketosen mit 5 oder 6 Kohlenstoffatomen, vorzugsweise von Glucose, ab. Die Indexzahl p in der allgemeinen Formel (II) gibt den Polymerisierungsgrad (DP), d. h. die Verteilung von Mono- und Polyglucosiden, an und steht für eine Zahl zwischen 1 und 10. Während p im einzelnen Molekül stets ganzzahlig sein muss und hier vor allem die Werte p = 1 bis 6 annehmen kann, ist der Wert p für ein bestimmtes Alkylpolyglucosid eine analytisch ermittelte rechnerische Größe. Vorzugsweise werden Alkylpolyglucoside mit einem mittleren Oligomerisierungsgrad p von 1,1 bis 3,0 eingesetzt. Aus anwendungstechnischer Sicht sind Alkylpolyglucoside bevorzugt, deren Polymerisierungsgrad (d.h. deren Zahl p) kleiner als 1,7 ist und insbesondere zwischen 1,2 und 1,4 liegt.

Der Alkylrest R1 in Formel (II) kann sich von primären Alkoholen mit 6 bis 11, vorzugsweise 8 bis 10 Kohlenstoffatomen ableiten. Typische Beispiele sind Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkylpolyglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkylpolyglucoside auf Basis technischer C_{9/11}-Oxoalkohole (DP = 1 bis 3).

Der Alkylrest R1 kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkylpolyglucoside auf Basis von gehärtetem C_{12/14}-Kokosalkohol mit einem DP von 1 bis 3.

Bevorzugt steht R1 für eine Cocosalkylgruppe, eine Stearylgruppe, eine Cetylgruppe, eine Laurylgruppe, Gemische aus C₈-C₁₀)-Alkylgruppen, Gemische aus C_{12/14}-Alkylgruppen und/oder deren Gemische.

In einer bevorzugten Ausführungsform des ersten Erfindungsgegenstands enthält das Mittel als nichtionisches Tensid (B) mindestens eine Verbindung der Formel (II), worin R1 für eine C₁₂-Alkylgruppe, eine C₁₄-Alkylgruppe oder eine Cocosalkylgruppe und G für einen Glucose-Rest stehen.

Erfindungsgemäß besonders geeignete Alkylpolyglucoside der Formel (II) werden unter der INCI-Bezeichnung Coco-Glucoside und dem Handelsnamen Plantacare 818 UP oder unter der INCI-Bezeichnung Lauryl-Glucoside und dem Handelsnamen Plantacare 1200 UP vertrieben.

Es ist bevorzugt, die Alkylpolyglucoside der Formel (II) in einem Anteil von 0,1 bis 12 Gew.-%, bevorzugt von 0,5 bis 10 Gew.-%, insbesondere bevorzugt von 1,0 bis 9 Gew.-% und weiter bevorzugt von 1,5 bis 7,5 Gew.-%, jeweils bezogen auf das Gesamtgewicht des Mittels, einzusetzen.

Als weiteren wesentlichen Inhaltsstoff enthält das erfindungsgemäße Mittel des ersten Erfindungsgegenstands schließlich mindestens ein kationisches Polymer (C), wobei als kationisches Polymer Polyquaternium-37 enthalten ist.

Unter kationischen Polymeren sind Polymere zu verstehen, welche in der Haupt- und/oder Seitenkette eine Gruppe aufweisen, welche "temporär" oder "permanent" kationisch vorliegt. Besonders vorteilhafte Effekte werden erzielt, wenn das kationische Polymer (C) in einer bestimmten Menge im Mittel eingesetzt wird, bevorzugt zumindest zu 0,05 Gew.-%. Eine weitere Ausführungsform des ersten Erfindungsgegenstands ist daher ein Mittel, welches dadurch gekennzeichnet ist, dass das Mittel kationische Polymere (C) in einem Anteil von 0,05 bis 7,5 Gew.-%, bevorzugt von 0,1 bis 5 Gew.-%, insbesondere von 0,12 bis 1,5 Gew.-% und weiter bevorzugt von 0,15 bis 0,75 Gew.-%, jeweils bezogen auf das Gesamtgewicht des Mittels, enthält.

Insbesondere im Hinblick auf Farbintensität, Haltbarkeit und Homogenität der Färbung sowie im Hinblick auf Kämmbarkeitsverbesserung ist es vorteilhaft, Fettkörper (A) und kationische und/oder amphotere Polymere (C) in einem bestimmten Verhältnis zueinander einzusetzen, vorteilhafterweise insbesondere etwa in gleichen Gewichtsanteilen.

Eine weitere Ausführungsform des ersten Erfindungsgegenstands ist daher ein Mittel, welches dadurch gekennzeichnet ist, dass es ein Gewichtsverhältnis aus Fettkörpern (A) und aus kationischen und/oder amphoteren Polymeren (C) mit einem Wert von 3 : 1 bis 1 : 3, bevorzugt 2 : 1 bis 1 :2, besonders bevorzugt 3 : 2 bis 2 : 3 aufweist.

Es kann erfindungsgemäß vorteilhaft sein, wenn das Mittel neben den Fettkörpern (A) gemäß Formel (I) zusätzliche Fettkörper enthält. Besonders vorteilhaft ist es, wenn das Mittel des ersten Erfindungsgegenstands zusätzlich mindestens einen zusätzlichen Fettkörper (A'), ausgewählt aus Fettsäurealkylestern, Fettalkylestern, Fettsäurefettalkylestern und Di-Fettalkylethern, enthält. Unter Fettsäurealkylester sind dabei erfindungsgemäß Carbonsäureester aus Fettsäuren und Mono- oder Polyhydroxy-C₁-C₄-Alkanolen zu verstehen.

Erfindungsgemäße Fettsäuren weisen 8 bis 22 Kohlenstoffatome auf und können einerseits verzweigte oder unverzweigte und andererseits gesättigte oder ungesättigte Alkylketten enthalten. Beispiele für Fettsäuren im Sinne der Erfindung sind
- gesättigte, unverzweigte Fettsäuren, wie Octansäure (Caprylsäure), Nonansäure (Pergalonsäure), Decansäure (Caprinsäure), Undecansäure, Dodecansäure (Laurinsäure), Tridecansäure, Tetradecansäure (Myristinsäure), Pentadecansäure, Hexadecansäure (Palmitinsäure), Heptadecansäure (Margarinsäure), Octadecansäure (Stearinsäure), Nonadecansäure, Eicosansäure (Arachinsäure), Docosansäure (Behensäure);
- gesättigte, verzweigte Fettsäuren, wie Isooctansäure, Isopalmitinsäure, Isostearinsäure;
- ungesättigte, unverzweigte Fettsäuren, wie Palmitoleinsäure ((9Z)-Hexadec-9-ensäure), Ölsäure ((9Z)-Octadec-9-ensäure), Elaidinsäure ((9E)-Octadec-9-ensäure), Erucasäure ((13Z)-Docos-13-ensäure), Linolsäure ((9Z,12Z)-Octadeca-9,12-diensäure), Linolensäure ((9Z,12Z,15Z)-Octadeca-9,12,15-triensäure), Elaeostearinsäure ((9Z,11E,13E)-Octadeca-9,11,13-triensäure), Arachidonsäure ((5Z,8Z,11Z,14Z)-Icosa-5,8,11, 14-tetraensäure).

Beispiele für Mono- oder Polyhydroxy-C₁-C₄-Alkanole sind Methanol, Ethanol, Propanol, Isopropanol, Ethylenglycol, Propylenglycol, Propan-1,3-diol, Butan-1,2-diol, Butan-1,3-diol, Butan-1,4-diol, Butan-2,3-diol und Glycerin.

Bevorzugte Beispiele für Fettsäurealkylester sind Isopropylmyristat, Isopropylpalmitat, Ethylenglycoldistearat sowie Fettsäuretriglyceride, insbesondere triglyceridische, pflanzliche Öle.

Unter Fettalkylestern sind dabei erfindungsgemäß Carbonsäureester aus Fettalkoholen und C₂-C₆-Mono- oder Dicarbonsäuren zu verstehen. Fettalkohole im Sinne der Erfindung sind terminale Alkohole, die eine gesättigte oder ungesättigte C₆-C₂₀-Alkylkette gemäß voranstehender Definition enthalten. Geeignete C₂-C₆-Mono- oder Dicarbonsäuren sind Essigsäure, Oxalsäure, Maleinsäure, Bernsteinsäure, Adipinsäure, Isobuttersäure und Propionsäure.

Beispiele für erfindungsgemäß bevorzugte Fettalkylester sind Di-(2-ethylhexyl)succinat, Di-octyladipat, Di-(2-ethylhexyl)maleat, 2-Ethylhexylacetat, Cetylacetat und Stearylacetat.

Unter Fettsäurefettalkylestern sind erfindungsgemäß Carbonsäureester aus Fettalkoholen und Fettsäuren gemäß voranstehender Definitionen zu verstehen.

Beispiele für erfindungsgemäß bevorzugte Fettsäurefettalkylester sind Myristylmyristat, Myristylpalmitat, Myristylstearat, Cetyl-(2-ethylhexanoat), Cetylpalmitat, Decyloleat, 2-Ethylhexylstearat, 2-Ethylhexyloleat und 2-Ethylhexylpalmitat.

Unter Di-Fettalkylethern sind erfindungsgemäß Dialkylether zu verstehen, bei denen beide Alkylreste unabhängig voneinander für eine gesättigte oder ungesättigte C₆-C₂₀-Alkylkette gemäß voranstehender Definition stehen. Bevorzugt sind C₆-C₁₄-Alkylketten.

Beispiele für erfindungsgemäß bevorzugte Di-Fettalkylethern sind Diheptylether, Dioctylether und Dilaurylether.

Im Falle eines weiteren Fettkörpers (A') sollte sich die Gesamtfettkörpermenge im Mittel vorzugsweise nicht erhöhen. Eine Ausführungsform des ersten Erfindungsgegenstands ist daher dadurch gekennzeichnet, dass das Mittel einen Gesamtanteil an Fettkörpern (A) und gegebenenfalls (A') von 0,05 bis 8,5 Gew.-%, bevorzugt von 0,1 bis 6 Gew.-%, insbesondere von 0,2 bis 4 Gew.-% und weiter bevorzugt von 0,3 bis 2,5 Gew.-%, jeweils bezogen auf das Gesamtgewicht des Mittels, aufweist.

Weiterhin zeigte sich, dass der Zusatz eines speziellen Aminoxid-Tensids zusätzliche Verbesserung im Hinblick auf Pflegeleistung und Kämmbarkeit bewirkt.

Eine weitere Ausführungsform des ersten Erfindungsgegenstands ist daher dadurch gekennzeichnet, dass das Mittel zusätzlich mindestens ein Aminoxid-Tensid der Formel (IV), 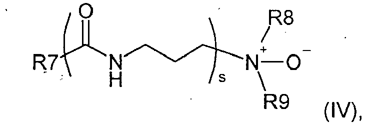 worin
- R7: für eine gesättigte oder ungesättigte C₆-C₂₂-Alkylkette steht,
- s: für die Zahl 0 oder 1 steht, und
- R8 und R9: jeweils unabhängig voneinander für Wasserstoff, eine C₁-C₄-Alkylkette oder eine C₂-C₄-Hydroxyalkylkette stehen,
enthält.

Hinsichtlich der gesättigten oder ungesättigten C₆-C₂₂-Alkylkette des Rests R7 gilt die vorstehende Definition. Bevorzugte Beispiele einer C₁-C₄-Alkylkette sind Methyl und Ethyl, n-Propyl oder i-Propyl, bevorzugt Methyl. Bevorzugte Beispiele einer C₂-C₄-Hydroxyalkylkette sind 2-Hydroxyethyl und 3-Hydroxypropyl.

Beispielhafte Verbindungen der Formel (IV) mit R8 = R9 = Wasserstoff sind die Verbindungen mit den INCI-Bezeichnungen Cocamine Oxide, Tallowamine Oxide, Decylamine Oxide, Lauramine Oxide, Myristamine Oxide, Palmitamine Oxide, Stearamine Oxide, Oleamine Oxide, Behenamine Oxide (je s = 0) sowie Cocamidopropylamine Oxide, Tallowamidopropylamine Oxide, Decylamidopropylamine Oxide, Lauramidopropylamine Oxide, Myristamidopropylamine Oxide, Palmitamidopropylamine Oxide, Stearamidopropylamine Oxide, Oleamidopropylamine Oxide, Behenamidopropylamine Oxide (je s = 1).

Beispielhafte Verbindungen der Formel (IV), in der R8 und R9 für eine C₂-C₄-Hydroxyalkylkette stehen, sind die Verbindungen mit den INCI-Bezeichnungen Dihydroxyethyl Cocamine Oxide, Dihydroxyethyl Tallowamine Oxide, Dihydroxyethyl Lauramine Oxide , Dihydroxyethyl Stearamine Oxide.

Bevorzugt stehen R8 und R9 jeweils für Methyl. Weiter bevorzugt steht s für 0. Beispielhafte und besonders bevorzugte Verbindungen der Formel (IV), in der R8 und R9 für Methyl stehen, sind die Verbindungen Dimethyl Cocamine Oxide, Dimethyl Tallowamine Oxide, Dimethyl Decylamine Oxide, Dimethyl Lauramine Oxide, Dimethyl Myristamine Oxide, Dimethyl Palmitamine Oxide, Dimethyl Stearamine Oxide, Dimethyl Oleamine Oxide und Dimethyl Behenamine Oxide sowie Dimethyl Cocamidopropylamine Oxide, Dimethyl Tallowamidopropylamine Oxide, Dimethyl Decylamidopropylamine Oxide, Dimethyl Lauramidopropylamine Oxide, Dimethyl Myristamidopropylamine Oxide, Dimethyl Palmitamidopropylamine Oxide und Dimethyl Stearamidopropylamine Oxide. Bevorzugt enthalten die erfindungsgemäßen Mittel Aminoxid-Tenside der Formel (IV) in einem Anteil von 0,01 bis 5 Gew.-%, bevorzugt 0,05 bis 2,5 Gew.-%, weiter bevorzugt 0,1 bis 1,5 Gew.-% und besonders bevorzugt 0,15 bis 1,0 Gew.-%, bezogen auf das Gesamtgewicht des Mittels.

Weiterhin hat es sich als vorteilhaft erweisen, wenn die Mittel mindestens einen Stabilisator oder Komplexbildner enthalten. Im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP) und Diethylentriaminpenta(methylenphosphonsäure) (DTPMP), Phösphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Die erfindungsgemäßen Mittel werden bevorzugt als fließfähige Zubereitungen formuliert. Dazu gehören insbesondere Emulsionen, Suspensionen und Gele, besonders bevorzugt Emulsionen. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und nichtionischen Tensiden ausgewählt sind.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen, bevorzugt 8 bis 24 C-Atomen. Zusätzlich können im Molekül Glykol- oder Polyglykolether-Gruppen, Ester-, Ether- und Amidgruppen sowie Hydroxylgruppen enthalten sein. Bevorzugte anionische Tenside sind Seifen, Alkylsulfate, Alkylethersulfate und Ethercarbonsäuren mit 10 bis 18 C-Atomen in Alkylgruppe und bis zu 12 Glykolethergruppen im Molekül. Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammonium-glycinate, N-Acyl-aminopropyl-N,N-dimethylammoniumglycinate und 2-Alkyl-3-carboxymethyl-3-hydroxyethyl-imidazoline sowie Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat. In einer weiteren Ausführungsform der vorliegenden Erfindung enthält das Mittel weiterhin mindestens ein amphoteres Tensid. Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine COOH- oder SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) und Bezeichnung Disodium Cocoamphodiacetate mit den Handelsnamen Dehyton (Cognis), Miranol C2M (Rhodia) und Ampholak XCO 30 (Akzo Nobel) vermarktet. Weiterhin hat es sich als vorteilhaft erwiesen, wenn die erfindungsgemäßen Färbe- oder Aufhellmittel nichtionogene grenzflächenaktive Stoffe enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Als zusätzliche weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten. Die anionischen, nichtionischen, amphoteren oder zwitterionischen Tenside werden in Gesamtmengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Alkylamidoamine. Bevorzugte quaternäre Ammoniumverbindungen sind Ammoniumhalogenide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside sind quaternisierte Proteinhydrolysate. Alkylamidoamine werden üblicherweise durch Amidierung natürlicher oder synthetischer Fettsäuren und Fettsäureschnitte mit Dialkylaminoaminen hergestellt. Eine erfindungsgemäß geeignete Verbindung aus dieser Substanzgruppe stellt Tegoamid® S 18 (Stearamidopropyldimethylamin) dar. Bei Esterquats handelt es sich um Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden unter den Warenzeichen Stepantex, Dehyquart und Armocare vertrieben. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Ferner können die erfindungsgemäßen Mittel weitere Wirk-, Hilfs- und Zusatzstoffe enthalten, wie beispielsweise Assoziativpolymere mit Fettalkylkette, nichtionische Polymere (wie Vinylpyrrolidinon/Vinylacrylat-Copolymere, Polyvinylpyrrolidinon, Vinylpyrrolidinon/Vinylacetat-Copolymere, Polyethylenglykole und Polysiloxane); anionische Polymere (wie Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert-Butyl-acrylamid-Terpolymere); Verdickungsmittel (Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol); haarkonditionierende Verbindungen (wie Phospholipide, wie Sojalecithin, Ei-Lecitin, Kephaline sowie Silikonöle); zusätzliche Proteinhydrolysate pflanzlicher oder tierischer Herkunft (wie Elastin-, Collagen-, Keratin-, Milcheiweiß-, Sojaprotein- und Weizenproteinhydrolysate, deren Kondensationsprodukte mit Fettsäuren sowie quaternisierte Proteinhydrolysate); Parfümöle, Dimethylisosorbid und Cyclodextrine; faserstrukturverbessernde Wirkstoffe (wie Mono-, Di- und Oligosaccharide, Glucose, Maleinsäure und Milchsäure); Entschäumer wie Silikone (Dimethicon); Farbstoffe zum Anfärben des Mittels; Antischuppenwirkstoffe (wie Piroctone Olamine, Zink Omadine und Climbazol); Lichtschutzmittel (wie derivatisierte Benzophenone, Zimtsäure-Derivate und Triazine); Wirkstoffe (Pantolacton, Allantoin, Pyrrolidinoncarbonsäuren und deren Salze sowie Bisabolol); Vitamine, Provitamine und Vitaminvorstufen, insbesondere solche der Gruppen A, B₃, B₅, B₆, C, E, F und H; Pflanzenextrakte; Cholesterin; Konsistenzgeber (wie Zuckerester, Polyolester oder Polyolalkylether); weitere Fette und Wachse (Fettalkohole, Bienenwachs, Montanwachs und Paraffine); Quell- und Penetrationsstoffe (Glycerin, Propylenglykolmonoethylether, Carbonate, Hydrogencarbonate, Guanidine, Harnstoffe sowie primäre, sekundäre und tertiäre Phosphate); Trübungsmittel (Latex, Styrol/PVP- und Styrol/Acrylamid-Copolymere); Perlglanzmittel (Ethylenglykolmonostearat sowie PEG-3-distearat); Treibmittel wie Propan-Butan-Gemische, N₂O, Dimethylether, CO₂ und Luft; Antioxidantien.

Die Auswahl dieser weiteren Stoffe wird der Fachmann gemäß den gewünschten Eigenschaften der Zubereitungen treffen. Die Zubereitungen enthalten die weiteren Wirk-, Hilfs- und Zusatzstoffe bevorzugt in Mengen von 0,01 bis 25 Gew.-%, insbesondere 0,05 bis 15 Gew.-%, bezogen auf die Gesamtmenge des anwendungsbereiten Mittel.

Die erfindungsgemäßen Mittel weisen bevorzugt einen pH-Wert im Bereich von 4 bis 12 auf. Bei den pH-Werten im Sinne der vorliegenden Erfindung handelt es sich um pH-Werte, die bei einer Temperatur von 22°C gemessen wurden.

Zur Einstellung des pH-Werts sind dem Fachmann gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basischen Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß einsetzbare, organische Alkalisierungsmittel werden bevorzugt ausgewählt aus Alkanolaminen, ausgewählt aus 2-Aminoethan-1-ol (Monoethanolamin), 2-Amino-2-methylpropan-1-ol, 2-Amino-2-methyl-propan-1,3-diol und Triethanolamin. Die basischen Aminosäuren werden bevorzugt ausgewählt aus Arginin und Lysin. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat, bevorzugt Natriumhydroxid und/oder Kaliumhydroxid. Schließlich ist ein weiteres bevorzugtes Alkalisierungsmittel Ammoniak.

Unter Container wird im Rahmen der vorliegenden Erfindung eine Umhüllung verstanden, die in Form einer gegebenenfalls wieder-verschließbaren Flasche, einer Tube, einer Dose, eines

Tütchens, eines Sachets oder ähnlichen Umhüllungen vorliegt. Dem Umhüllungsmaterial sind erfindungsgemäß keine Grenzen gesetzt. Bevorzugt handelt es sich jedoch dabei um Umhüllungen aus Glas oder Kunststoff..

Die erfindungsgemäßen Mittel können in Verfahren zur Farbveränderung menschlicher Haare genutzt werden. Dabei wird ein erfindungsgemäßes Mittel auf das Haar aufgetragen und für einen Zeitraum von 3 bis 45 Minuten, bevorzugt 5 bis 30 Minuten im Haar belassen. Anschließend wird das Haar mit Wasser und/oder einem handelsüblichen Shampoo gespült.

Die Auftragungs- und die Einwirktemperatur der Farbveränderungszubereitung beträgt Raumtemperatur bis 45°C. Gegebenenfalls kann die Wirkung der Farbveränderungszubereitung durch externe Wärmezufuhr, wie beispielsweise mittels einer Wärmehaube, verstärkt werden. Die bevorzugte Einwirkdauer der Farbveränderungszubereitung auf die keratinische Faser beträgt 3 bis 45 Minuten, bevorzugt 5 bis 30 Minuten. Nach Beendigung der Einwirkdauer wird das verbliebene Farbveränderungsmittel aus den keratinischen Fasern mit Hilfe einer Reinigungszubereitung oder Wasser ausgewaschen. Nach dem Auswaschen werden die keratinischen Fasern gegebenenfalls mit einem Handtuch oder einem Heißluftgebläse getrocknet. Die Auftragung der Farbveränderungszubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Farbveränderungsmittel mit einer Applikationshilfe auf die keratinischen Fasern aufzutragen.

Durch Verwendung der erfindungsgemäßen Mittel auf menschlichen Haaren lassen sich bei der Färbung menschlicher Haare Haarpflege verbessern und/oder Kämmbarkeit verbessern und/oder Farbintensität steigern und/oder Farberhalt, insbesondere gegenüber Haarwäschen, verbessern.

### Beispiele

### 1. Formulierungen

### (soweit nicht anders angegeben entsprechen die Mengenangaben Gewichtsanteilen)

### 1.1 Herstellung der nicht erfindungsgemäßen Färbezubereitung (Tabelle 1)

| Rohstoff | **A** | **B** | **C** | **D** | **E** | **V1** | **V2** |
|---|---|---|---|---|---|---|---|
| Ammoniumcarbomer | 0,12 | 0,12 | 0,12 | 0,12 | 0,12 | 0,12 | 0,15 |
| Natrium Cetearyl Sulfate | 0,56 | 0,56 | 0,56 | 0,56 | 0,56 | 0,56 | 0,70 |
| Natrium Laureth Sulfate | - | - | - | - | - | 0,95 | 1,19 |
| Kaliumoleat | 0,30 | 0,30 | 0,30 | 0,30 | 0,30 | 0,30 | 0,38 |
| Lamesoft PO 65 | 1,50 | 1,50 | 1,50 | - | - | - | - |
| Aramox MCD W | - | - | - | 1,50 | 1,50 | - | - |
| Cutina GMS SE | 3,00 | 3,00 | 3,00 | 3,00 | 3,00 | 1,60 | 4,00 |
| Ethylenglycol Distearate | - | - | - | - | - | 1,60 | - |
| 2-Octyldodecanol | - | - | - | - | - | 1,60 | 2,00 |
| Dioctylcarbonat | 1,60 | - | - | - | - | - | - |
| Dioctylether | - | 1,60 | - | - | 1,60 | - | - |
| Cetyl 2-ethylhexanoat | - | - | 1,60 | 1,60 | - | - | - |
| Cetearvlalkohol | 9,90 | 9,90 | 9,90 | 9,90 | 9,90 | 9,60 | 12,00 |
| Ceteareth-20 | 2,40 | 2,40 | 2,40 | 2,40 | 2,40 | 2,40 | 3,00 |
| Phospholipid EFA | 0,10 | - | - | - | - | 0,1 | 0,1 |
| Triple C | 0,70 | 0,70 | 0,70 | 0,70 | 0,70 | - | - |
| L-Serin | 0,50 | 0,50 | 0,50 | 0,50 | 0,50 | 0,50 | 0,50 |
| EDTA, Na₂ | 0,20 | 0,20 | 0,20 | 0,20 | 0,20 | 0,20 | 0,20 |
| Merquat Plus 3330 | - | - | - | - | - | - | 1,50 |
| p-Toluylendiaminsulfat | 0,84 | 0,84 | 0,84 | 0,84 | 0,84 | 0,84 | 0,84 |
| 4-Chlorresorcin | 0,13 | 0,13 | 0,13 | 0,13 | 0,13 | 0,13 | 0,13 |
| 3-Aminophenol | 0,10 | 0,10 | 0,10 | 0,10 | 0,10 | 0,10 | 0,10 |
| Resorcin | 0,23 | 0,23 | 0,23 | 0,23 | 0,23 | 0,23 | 0,23 |
| Ethanolamin | - | - | - | - | - | - | 0,30 |
| Kaliumhydroxid | - | - | - | - | - | 0,35 | - |
| Ascorbinsäure | - | - | - | - | - | 0,05 | 0,05 |
| Natriumsulfit | 0,25 | 0,25 | 0,25 | 0,25 | 0,25 | 0,15 | 0,15 |
| Ammoniak, 25 Gew.-%, wässrig | 6,00 | 6,00 | 6,00 | 6,00 | 6,00 | 6,00 | 6,00 |
| Wasser | Ad 100 | | | | | | |

### 1.2 Herstellung der Oxidationsmittelzubereitung (Tabelle 2)

| Rohstoff | **II-1** | **II-2** |
|---|---|---|
| Phosphorsäure, 85 Gew.-% | 0,04 | - |
| EDTA, Na₂ | 0,15 | - |
| Dinatriumpyrophosphat | 0,30 | 0,10 |
| Natrium Benzoat | 0,04 | 0,04 |
| Emulgade F | 2,20 | - |
| Dipicolinsäure | - | 0,10 |
| Kaliumhydroxid | - | 0,10 |
| 1,2-Propandiol | - | 0,50 |
| Etidronic Acid | - | 0,15 |
| Paraffinum Liquidum | - | 2,00 |
| Cetearyl Alkohol | - | 3,40 |
| Ceteareth-20 | - | 1,00 |
| Wasserstoffperoxid, 50 Gew.-%, wässrig | 12,00 | 12,20 |
| Wasser | ad 100 | |

- Lamesoft PO 65: Aktivsubstanzgehalt ca. 64-68%; INCI-Bezeichnung: Coco-Glucoside (20-40%), Glyceryl Oleate (20-40%), Aqua
- Aramox MCD W: Aktivsubstanzgehalt ca. 30%; INCI-Bezeichnung: Dimethyl Cocamine Oxide
- Cutina GMS SE: Aktivsubstanzgehalt ca. 32-36%; INCI-Bezeichnung: Glyceryl Stearate SE
- Phospholipid EFA: Aktivsubstanzgehalt ca. 30%; INCI-Bezeichnung: Linoleamidopropyl PG-Dimonium Chloride Phosphate, Propylene Glycol (25%)
- Merquat Plus 3330: Aktivsubstanzgehalt ca.10%; INCI-Bezeichnung: Polyquaternium-39
- Triple C: INCI-Bezeichnung: Polyquaternium-37 (ca. 50 %), Dioctylcarbonat (ca. 48%), Lauryl Glucoside (ca. 2 %)
- Emulgade F: INCI-Bezeichnung: Cetearyl Alcohol (70-85%), PEG-40 Castor Oil (10-20%), Sodium Cetearyl Sulfate (5-10%)

### 2. Anwendung

Färbezubereitung (I) und Oxidationszubereitung (II-1) wurden zu gleichen Gewichtsanteilen zu einem anwendungsbereiten Färbemittel vermischt. Für den Färbeprozess wurde auf Haarsträhnen (weiß, Euronaturhaar Weiß (Kerling ENHw)) von ca. 0,7 g Gewicht jeweils die 4-fache Menge dieser Anwendungsmischung appliziert. Nach einer Einwirkzeit von 30 Minuten bei 32 °C wurden die Strähnen mit Wasser ausgewaschen und mit einem Handtuch getrocknet.

Die mit den nicht erfindungsgemäßen Mitteln A bis E gefärbten Strähnen wiesen eine verbesserte Kämmbarkeit und einen besseren Griff im Nassen und trockenen Zustand gegenüber der mit der Vergleichszubereitung V1 gefärbten Strähne auf.

Im Halbseitenversuch wurden Probanden auf der einen Hälfte des Kopfhaars mit einer Anwendungsmischung aus Vergleichszubereitung V2 und Oxidationszubereitung (II-2) und auf der anderen Hälfte mit einer Anwendungsmischung aus dem erfindungsgemäßen Mittel D und Oxidationszubereitung (II-1) unter gleichen Bedingungen gefärbt. Die beiden Hälften wurden hinsichtlich ihres Pflegezustands und ihrer Kämmbarkeit von Friseuren begutachtet und miteinander verglichen.

Die mit dem nicht erfindungsgemäßen Mittel D gefärbte Hälfte wies einen verbesserten Pflegezustand auf, der sich in einer leichteren Kämmbarkeit und einen verbesserten Griff im nassen und trockenen Zustand darstellte.

## Patentansprüche

1. Mittel zum Färben keratinischer Fasern, enthaltend in einem kosmetisch akzeptablen Träger mindestens einen direktziehenden Farbstoff, **dadurch gekennzeichnet, dass** das Mittel eine Trägerbasis-Kombination aus
(i) mindestens einem Fettkörper (A) der Formel (I), 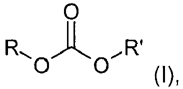 worin R und R' jeweils unabhängig voneinander für eine gesättigte oder ungesättigte C₆-C₂₂-Alkylkette stehen,
(ii) mindestens einem nichtionischen Tensid (B) der Formel (II),
R1-O-[G]ₚ (II),
worin R1 für eine gesättigte oder ungesättigte C₆-C₂₂-Alkylkette, G für einen Zuckerrest mit 5 oder 6 Kohlenstoffatomen und p für eine Zahl von 1 bis 10 stehen und
(iii) mindestens einem kationischen Polymer (C)
enthält,
**dadurch gekennzeichnet, dass** das Mittel als kationisches Polymer (C) Polyquaternium-37 enthält.

2. Mittel nach Anspruch 1, **dadurch gekennzeichnet, dass** es als Fettkörper (A) der Formel (I) Di-n-octylcarbonat und/oder Di-(2-Ethylhexyl)carbonat enthält.

3. Mittel nach einem der Ansprüche 1 oder 2, **dadurch gekennzeichnet, dass** es als nichtionisches Tensid (B) mindestens eine Verbindung der Formel (II) enthält, worin R1 für eine C₁₂-Alkylgruppe, eine C₁₄-Alkylgruppe oder eine Cocosalkylgruppe und G für einen Glucose-Rest stehen.

4. Mittel nach einem der Ansprüche 1 bis 3, **dadurch gekennzeichnet, dass** die Alkylpolyglucoside der Formel (II) in einem Anteil von 0,1 bis 12 Gew.-%, bevorzugt von 0,5 bis 10 Gew.-%, insbesondere bevorzugt von 1,0 bis 9 Gew.-% und weiter bevorzugt von 1,5 bis 7,5 Gew.-%, jeweils bezogen auf das Gesamtgewicht des Mittels, enthalten sind.

5. Mittel nach einem der Ansprüche 1 bis 4, **dadurch gekennzeichnet, dass** das Mittel kationische Polymere (C) in einem Anteil von 0,05 bis 7,5 Gew.-%, bevorzugt von 0,1 bis 5 Gew.-%, insbesondere von 0,12 bis 1,5 Gew.-% und weiter bevorzugt von 0,15 bis 0,75 Gew.-%, jeweils bezogen auf das Gesamtgewicht des Mittels, enthält.

6. Mittel nach einem der Ansprüche 1 bis 5, **dadurch gekennzeichnet, dass** es ein Gewichtsverhältnis aus Fettkörpern (A) und aus kationischen Polymeren (C) mit einem Wert von 3 : 1 bis 1 : 3, bevorzugt 2 : 1 bis 1 :2, besonderes bevorzugt 3 : 2 bis 2 : 3 aufweist.

7. Mittel nach einem der Ansprüche 1 bis 6, **dadurch gekennzeichnet, dass** das Mittel zusätzlich mindestens einen zusätzlichen Fettkörper (A'), ausgewählt aus Fettsäurealkylestern, Fettalkylestern, Fettsäurefettalkylestern und Di-Fettalkylethern, enthält.

8. Mittel nach einem der Ansprüche 1 bis 7, **dadurch gekennzeichnet, dass** das Mittel einen Gesamtanteil an Fettkörpern (A) und gegebenenfalls (A') von 0,05 bis 8,5 Gew.-%, bevorzugt von 0,1 bis 6 Gew.-%, insbesondere von 0,2 bis 4 Gew.-% und weiter bevorzugt von 0,3 bis 2,5 Gew.-%, jeweils bezogen auf das Gesamtgewicht des Mittels, aufweist.

9. Mittel nach einem der Ansprüche 1 bis 8, **dadurch gekennzeichnet, dass** das Mittel zusätzlich mindestens ein Aminoxid-Tensid der Formel (IV), 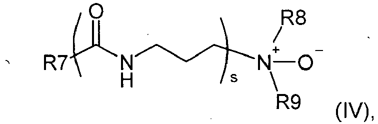 worin
R7 für eine gesättigte oder ungesättigte C₆-C₂₂-Alkylkette steht,
s für die Zahl 0 oder 1 steht, und
R8 und R9 jeweils unabhängig voneinander für Wasserstoff, eine C₁-C₄-Alkylkette oder eine C₂-C₄-Hydroxyalkylkette stehen,
enthält.

10. Mittel nach einem der Ansprüche 1 bis 9, **dadurch gekennzeichnet, dass** es mindestens eine Verbindung der Formel (IV), in der R8 und R9 für Methyl stehen, enthält, die ausgewählt ist aus Dimethyl Cocamine Oxide, Dimethyl Tallowamine Oxide, Dimethyl Decylamine Oxide, Dimethyl Lauramine Oxide, Dimethyl Myristamine Oxide, Dimethyl Palmitamine Oxide, Dimethyl Stearamine Oxide, Dimethyl Oleamine Oxide und Dimethyl Behenamine Oxide sowie Dimethyl Cocamidopropylamine Oxide, Dimethyl Tallowamidopropylamine Oxide, Dimethyl Decylamidopropylamine Oxide, Dimethyl Lauramidopropylamine Oxide, Dimethyl Myristamidopropylamine Oxide, Dimethyl Palmitamidopropylamine Oxide und Dimethyl Stearamidopropylamine Oxide.

11. Mittel nach einem der Ansprüche 1 bis 10, **dadurch gekennzeichnet, dass** es Aminoxid-Tenside der Formel (IV) in einem Anteil von 0,01 bis 5 Gew.-%, bevorzugt 0,05 bis 2,5 Gew.-%, weiter bevorzugt 0,1 bis 1,5 Gew.-% und besonders bevorzugt 0,15 bis 1,0 Gew.-%, bezogen auf das Gesamtgewicht des Mittels, enthält.

## Claims

1. An agent for dyeing keratin fibers, containing, in a cosmetically acceptable carrier, at least one direct dye, **characterized in that** the agent contains a carrier base combination of
(i) at least one fatty substance (A) of formula (I), 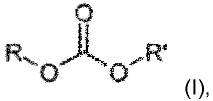 where R and R' each represent, independently of one another, a saturated or unsaturated C₆-C₂₂ alkyl chain,
(ii) at least one non-ionic surfactant (B) of formula (II),
R1-O-[G]ₚ (II),
where R1 represents a saturated or unsaturated C₆-C₂₂ alkyl chain, G represents a sugar residue having 5 or 6 carbon atoms, and p represents a number from 1 to 10, and
(iii) at least one cationic polymer (C),
**characterized in that** the agent contains polyquaternium-37 as a cationic polymer (C).

2. The agent according to claim 1, **characterized in that** it contains di-n-octyl carbonate and/or di(2-ethylhexyl) carbonate as a fatty substance (A) of formula (I).

3. The agent according to one of claims 1 or 2, **characterized in that** it contains at least one compound of formula (II) as a non-ionic surfactant (B), where R1 represents a C₁₂ alkyl group, a C₁₄ alkyl group or a coconut alkyl group, and G represents a glucose functional group.

4. The agent according to one of claims 1 to 3, **characterized in that** the alkyl polyglucosides of formula (II) are contained in a proportion of from 0.1 to 12 wt.%, preferably from 0.5 to 10 wt.%, particularly preferably from 1.0 to 9 wt.%, and more preferably from 1.5 to 7.5 wt.%, in each case based on the total weight of the agent.

5. The agent according to one of claims 1 to 4, **characterized in that** the agent contains cationic polymers (C) in a proportion of from 0.05 to 7.5 wt.%, preferably from 0.1 to 5 wt.%, in particular from 0.12 to 1.5 wt.%, and more preferably from 0.15 to 0.75 wt.%, in each case based on the total weight of the agent.

6. The agent according to one of claims 1 to 5, **characterized in that** it has a weight ratio of fatty substances (A) to cationic polymers (C) which has a value of from 3:1 to 1:3, preferably from 2:1 to 1:2, particularly preferably from 3:2 to 2:3.

7. The agent according to one of claims 1 to 6, **characterized in that** the agent additionally contains at least one additional fatty substance (A'), selected from fatty acid alkyl esters, fatty alkyl esters, fatty acid fatty alkyl esters and di-fatty alkyl ethers.

8. The agent according to one of claims 1 to 7, **characterized in that** the agent has a total proportion of fatty substances (A) and optionally (A') of from 0.05 to 8.5 wt.%, preferably from 0.1 to 6 wt.%, in particular from 0.2 to 4 wt.%, and more preferably from 0.3 to 2.5 wt.%, in each case based on the total weight of the agent.

9. The agent according to one of claims 1 to 8, **characterized in that** the agent additionally contains at least one amine oxide surfactant of formula (IV), 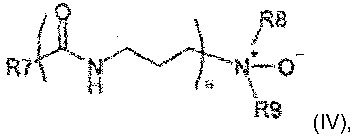 where
R7 represents a saturated or unsaturated C₆-C₂₂ alkyl chain,
s represents the number 0 or 1, and
R8 and R9 each represent, independently of one another, hydrogen, a C₁-C₄ alkyl chain or a C₂-C₄ hydroxyalkyl chain.

10. The agent according to one of claims 1 to 9, **characterized in that** it contains at least one compound of formula (IV), where R8 and R9 represent methyl, which compound is selected from dimethyl cocoamine oxide, dimethyl tallowamine oxide, dimethyl decylamine oxide, dimethyl lauramine oxide, dimethyl myristamine oxide, dimethyl palmitamine oxide, dimethyl stearamine oxide, dimethyl oleamine oxide, dimethyl behenamine oxide, dimethyl cocamidopropylamine oxide, dimethyl tallowamidopropylamine oxide, dimethyl decylamidopropylamine oxide, dimethyl lauramidopropylamine oxide, dimethyl myristamidopropylamine oxide, dimethyl palmitamidopropylamine oxide and dimethyl stearamidopropylamine oxide.

11. The agent according to one of claims 1 to 10, **characterized in that** it contains amine oxide surfactants of formula (IV) in a proportion of from 0.01 to 5 wt.%, preferably from 0.05 to 2.5 wt.%, more preferably from 0.1 to 1.5 wt.%, and particularly preferably from 0.15 to 1.0 wt.%, based on the total weight of the agent.

## Revendications

1. Agent de coloration de fibres kératiniques, contenant dans un support cosmétique acceptable cosmétiquement au moins un colorant à action directe, **caractérisé en ce que** l'agent contient une combinaison de base de support issue de
(i) au moins un corps gras (A) de la formule (I), 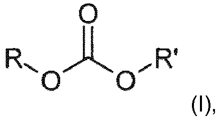 dans laquelle
R et R' représentent respectivement indépendamment l'un de l'autre une chaîne alkyle en C₆-C₂₂ saturée ou insaturée,
(ii) au moins un tensioactif non-ionique (B) de la formule (II),
R1-O-[G]ₚ (II),
dans laquelle R1 représente une chaîne alkyle en C₆-C₂₂ saturée ou insaturée, G représente un résidu de sucre comportant 5 ou 6 atomes de carbone et p représente un chiffre allant de 1 à 10 et
(iii)au moins un polymère cationique (C),
**caractérisé en ce que** l'agent contient le polyquaternium-37 en tant que polymère cationique (C).

2. Agent selon la revendication 1, **caractérisé en ce qu'**il contient du carbonate de di-n-octyle et/ou du carbonate de di-(2-éthylhexyl) en tant que corps gras (A) de la formule (I).

3. Agent selon l'une des revendications 1 ou 2, **caractérisé en ce qu'**il contient, en tant que tensioactif (B), au moins un composé de la formule (II), dans laquelle R1 représente un groupe alkyle en C₁₂, un groupe alkyle en C₁₄ ou un groupe alkyl de coco et G représente un résidu de glucose.

4. Agent selon l'une des revendications 1 à 3, **caractérisé en ce que** les alkylpolyglucosides de la formule (II) sont présents dans une proportion allant de 0,1 à 12 % en poids, de préférence de 0,5 à 10 % en poids, de manière particulièrement préférée de 1,0 à 9 % en poids et de manière davantage préférée de 1,5 à 7,5 % en poids, respectivement rapporté au poids total de l'agent.

5. Agent selon l'une des revendications 1 à 4, **caractérisé en ce que** l'agent contient des polymères cationiques (C) dans une proportion allant de 0,05 à 7,5 % en poids, de préférence de 0,1 à 5 % en poids, en particulier de 0,12 à 1,5 % en poids et de manière davantage préférée de 0,15 à 0,75 % en poids, respectivement rapporté au poids total de l'agent.

6. Agent selon l'une des revendications 1 à 5, **caractérisé en ce qu'**il présente un rapport de poids en corps gras (A) et en polymères cationiques (C) d'une valeur de 3:1 à 1:3, de préférence de 2:1 à 1:2, de manière particulièrement préférée de 3:2 à 2:3.

7. Agent selon l'une des revendications 1 à 6, **caractérisé en ce que** l'agent contient de plus au moins un corps gras supplémentaire (A'), sélectionné parmi les esters alkyliques d'acide gras, les esters alkyliques gras, les esters alkyliques gras d'acide gras et les diéthers alkyliques gras.

8. Agent selon l'une des revendications 1 à 7, **caractérisé en ce que** l'agent présente une proportion totale en corps gras (A) et le cas échéant (A') allant de 0,05 à 8,5 % en poids, de préférence de 0,1 à 6 % en poids, en particulier de 0,2 à 4 % en poids, et de manière davantage préférée de 0,3 à 2,5 % en poids, respectivement rapporté au poids total de l'agent.

9. Agent selon l'une des revendications 1 à 8, **caractérisé en ce que** l'agent contient de plus au moins un tensioactif d'aminoxyde de la formule (IV), 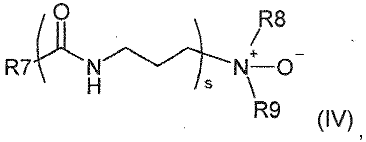 dans laquelle
R7 représente une chaîne alkyle en C₆-C₂₂ saturée ou insaturée,
s représente le chiffre 0 ou 1, et
R8 et R9 représentent respectivement indépendamment l'un de l'autre de l'hydrogène, une chaîne alkyle en C1-C4 ou une chaîne hydroxyalkyle en C₂-C₄.

10. Agent selon l'une des revendications 1 à 9, **caractérisé en ce qu'**il contient au moins un composé de la formule (IV), dans laquelle R8 et R9 représentent du méthyle, qui est sélectionné parmi l'oxyde de cocamine diméthylique, l'oxyde d'amine de suif diméthylique, l'oxyde de décylamine diméthylique, l'oxyde de lauramine diméthylique, l'oxyde de myristamine diméthylique, l'oxyde de palmitamine diméthylique, l'oxyde de stéaramine diméthylique, l'oxyde d'oléamine diméthylique et l'oxyde de béhénamine diméthylique, ainsi que l'oxyde de cocamidopropylamine diméthylique, l'oxyde d'amidopropylamine de suif diméthylique, l'oxyde de décylamidopropylamine diméthylique, l'oxyde de lauramidopropylamine diméthylique, l'oxyde de myristamidopropylamine diméthylique, l'oxyde de palmitamidopropylamine diméthylique et l'oxyde de stéaramidopropylamine diméthylique.

11. Agent selon l'une des revendications 1 à 10, **caractérisé en ce qu'**il contient des tensioactifs d'aminoxyde de la formule (IV) dans une proportion allant de 0,01 à 5 % en poids, de préférence de 0,05 à 2,5 % en poids, de manière davantage préférée de 0,1 à 1,5 % en poids et de manière particulièrement préférée de 0,15 à 1,0 % en poids, rapporté au poids total de l'agent.
